# EUROPEAN PATENT APPLICATION

(11) **EP 4 071 243 A1**
(43) Date of publication of application: **12.10.2022**
(21) Application number: 21167407.2
(22) Date of filing: 08.04.2021
(51) Int. Cl.: C12N 9/42, C12N 1/14, C12N 15/80

(54) **PROCESS FOR THE PRODUCTION OF A TECHNICAL ENZYME COMPOSITION WITH LOW VISCOSITY PRODUCED BY A FILAMENTOUS FUNGUS**

(71) Applicant: Clariant Produkte (Deutschland) GmbH, 65929 Frankfurt am Main (DE)
(72) Inventor: GAMAUF, Christian, 81241 München (DE); CLAREN, Jörg, 82131 Stockdorf (DE)
(74) Representative: Graser, Konstanze

(57) **Abstract**

The present invention relates to a process for the production of a technical enzyme composition with low viscosity, a genetically modified filamentous fungus cell suitable for production of the technical enzyme composition, the use of such a genetically modified filamentous fungus cell for the production of the technical enzyme composition with low viscosity and a technical enzyme composition with low viscosity produced by such a process.

## Description

The present invention relates to a process for the production of a technical enzyme composition with low viscosity produced by a genetically modified filamentous fungus cell, a genetically modified filamentous fungus cell suitable for production of the technical enzyme composition, the use of such a genetically modified filamentous fungus cell for the production of the technical enzyme composition with low viscosity and a technical enzyme composition with low viscosity produced by such a process.

Enzymes are important components of many commercial products and respective production processes. Modern laundry compositions contain a wide variety of different enzymes such as cellulases, many feed products for livestock contain enzymes and enzymes are also used for the production of many commercial products such as the production of bioethanol, of plastic alternatives / biodegradable plastics or even food products. Enzymes used in such processes are often called "industrial enzymes" or "technical enzymes".

To attain economic feasibility of the desired end product, a high yield and low production cost of the used technical enzyme(s) is a necessity. This applies in particular when the desired commercial end product is a bulk product which has to compete with low price alternatives originating from cheap mineral-oil derived chemical synthesis processes.

Filamentous fungi are well known as effective producers of a wide variety of technically feasible enzymes. In addition, filamentous fungi are able to grow on a diverse range of substrates.

However, the implementation of filamentous fungi for the production of technical enzymes is still not very popular as the high viscosity of the fermentation broth of such fungi often affords time and cost consuming measures leading to too high production costs of the technical enzyme composition. In order to obtain a high yield of enzymes, a strong growth of the fungus is desired, however, strong growth results in a high content of fungus biomass within the fermentation broth. Fungi, which are known to consist of *i.a.* hyphae are known within the art as rendering any fermentation substrate into a high-viscous composition. This effect is significantly more distinct when a filamentous fungus is used which exhibits a sponge-like, slimy appearance.

High viscosity causes many problems, as the fungus needs constant oxygen supply by aeration during growth. In addition, cooling of the fermenter, especially in industrial-scale production is required. Both can only be guaranteed by constant stirring - on the one hand to distribute the air bubbles homogenously within the broth, and on the other hand to facilitate constant heat-exchange with the cooling devices. The higher the viscosity of the broth the more energy needs to be spent to realize effective stirring within the reactor. Further, more air has to be pressed into the reactor causing also higher energy consumption within the compressor and sterile-filter unit. Thus, both CAPEX and OPEX increase with increasing viscosity of the fermentation broth. An alternative measure - less cell mass production - is also not attractive for commercial production as this would always be accompanied by a lower yield of technical enzyme production.

The inventors of the present invention have therefore set themselves the task to develop a process for the production of a technical enzyme composition with low viscosity produced by a filamentous fungus while maintaining a high yield of enzymes.

The task has been solved by a process for production of a technical enzyme composition, comprising the following steps:
(a) providing a fermentation medium with a glucose content of from 5 to 450 g/L;
(b) addition of at least one filamentous fungus cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

It is of particular advantage of the inventive process that a high yield of target enzymes is achieved with any kind of medium which contains a significant amount of glucose. The majority of the proteins secreted by filamentous fungi are enzymes that degrade naturally occurring polymers such as cellulose and hemicellulose and the availability of glucose would usually prevent the filamentous fungus from producing such enzymes as they are not needed for metabolization of glucose. Further, no addition of expensive inducing substances such as gluco-oligosaccharides or sophorose is necessary. Therefore, a wide variety of different fermentation substrates which are readily and cheaply available may be used.

Within the present invention the term "technical enzyme composition" is to be understood to consist of or to contain a partly or completely fermented medium and may even contain components of the original medium but also any compound generated during the fermentation process such as enzymes. A "technical enzyme composition" may also contain part of or all of the microbial biomass of the fermentation microorganism i.e. the filamentous fungus.

Within the present invention the technical enzyme composition preferably contains at least one enzyme belonging to the class of hydrolases and/or at least one enzyme belonging to the class of oxidoreductases. Within a particularly preferred embodiment of the present invention, the technical enzyme composition contains at least one enzyme belonging to the class of hydrolases and/or at least one enzyme belonging to the class of oxidoreductases which has been produced by the at least one filamentous fungus cell. Within another also particularly preferred embodiment, the technical enzyme composition contains at least one enzyme belonging to the class of cellulases and/or at least one enzyme belonging to the class of hemicellulases which has been produced by the at least one filamentous fungus cell.

Within the present invention, the term "enzyme belonging to the class of hydrolases" is to be understood as comprising any enzyme, capable of the hydrolysis of a chemical bond. Enzymes belonging to the class of hydrolases are classified as EC 3 in the EC number classification of enzymes. According to the present invention, the term "hydrolases" comprises cellulases, hemicellulases and may also encompass pectinases, oxidases, chitinases, chitosanases, transglutaminases, pentosanases, niringinases, limoninases, lactonases, nucleases, ureases, lipoxygenases, esterases, alpha-glucanases, phosphatases, isomerases, proteases and accessory proteins.

Within the present invention, the "enzyme belonging to the class of hydrolases" may be a native enzyme of the filamentous fungus or a heterologous enzyme originating from a different species of microorganism, in particular from a different species of filamentous fungus but may also originate from a non-filamentous fungus or a bacterium.

As used within the present invention, the term "cellulase" refers to any enzyme capable of hydrolyzing cellulose polymers to shorter oligomers and/or glucose. Cellulases preferred within the technical enzyme composition include cellobiohydrolases (CBH) (EC 3.2.1.-), endo-1,4-β-glucanases (EG) (EC 3.2.1.4).), beta-glucosidase (EC 3.2.1.4), cellobiose hydrolase (EC 3.2.1.21), glycoside hydrolase 61 (GH61 and CBM33).

As used within the present invention, the term "hemicellulase" refers to any enzyme capable of degrading or supporting the degradation of hemicellulose. Hemicellulases preferred within the technical enzyme composition include β-glucanases (EC 3.2.1.-), endo-xylanases (EC 3.2.1.8), β-xylosidases (EC 3.2.1.37), acetylxylan esterase (EC 3.1.1.72), acetylgalactan esterase (3.1.1.6), acetyl mannan esterase, feruloyl esterase (EC 3.1.1.73), glucuronoyl esterase (EC 3.1.1.-), α-L-arabinofuranosidase (EC 3.2.1.55), α-arabinopyranosidase (3.2.1.-), α-galactosidase (EC 3.2.1.22), β-galactosidase (EC 3.2.1.23), a-glucuronidases (EC 3.2.1.139), β-mannase (EC 3.2.1.78), β-mannosidases (EC 3.2.1.25), mannan 1,4-mannobiosidase (EC 3.2.1.100), arabinogalactan endo-beta-1,4-galactanase (EC 3.2.1.89), endo-beta-1,3-galactanase (EC 3.2.1.90), galactan endo-beta-1,3-galactanase (EC 3.2.1.181, glucuronoarabinoxylan endo-1,4-beta-xylanase (EC 3.2.1.136), alpha-L-fucosidase (EC 3.2.1.51), coniferin beta-glucosidase (EC 3.2.1.126), xyloglucan hydrolases (EC 3.2.1.150, 151, 155), xylan α-1,2-glucuronosidase (EC 3.2.1.131), endo-xylogalacturonan hydrolase (EC 3.2.1.-; GH28), α-amylase (EC 3.2.1.1), glucan 1,4-α-glucosidase (EC 3.2.1.3), galactan 1,3-galactosidase (GH43), -1,4,-endogalactanase (EC 3.5.1.89; GH53), α-rhamnosidase (EC 3.2.1.40) and β-rhamnosidase (EC 3.2.1.43).

As used within the present invention, the term "pectinase" refers to any enzyme capable of degrading or supporting the degradation of pectin. Pectinases preferred within the technical enzyme composition include polygalacturonases (EC 3.2.1.15, 67, 82; GH28 pectin methyl esterase (EC 3.1.1.11), pectin acetyl esterase (EC 3.1.1.-), rhamnogalacturonase (EC 3.2.1.-; GH28), rhamnogalacturonan acetylesterase (EC 3.1.1.86), rhamnogalacturonan galacturonohydrolase (EC 3.2.1.-), xylogalacturonan hydrolase (EC 3.2.1.-), pectin methylesterase (EC 3.1.1.11), beta-arabinofuranosidase (EC 3.2.1.55), beta-1,4-galactanase (EC 3.2.1.89), beta-1,3-galactanase (EC 3.2.1.90), beta-galactosidase (EC 3.2.1.23), alpha-galactosidase (EC 3.2.1.22), feruloyl acetyl esterase (EC 3.1.1.-), alpha-fucosidase (EC 3.2.1.51), (beta-fucosidase) (EC 3.2.1.38), beta-apiosidase (EC 3.2.1.-), alpha-rhamnosidase (EC 3.2.1.40), beta-rhamnosidase (EC 3.2.1.43), alpha-arabinopyranosidase (EC 3.2.1.-), beta-glucuronidase (EC 3.2.1.31), alpha-glucuronidase (EC 3.2.1.139), beta-xylosidase (EC 3.2.1.37) and alpha-xylosidase (EC 3.2.1.x).

As used within the present invention the term "accessory protein" refers to any enzyme capable of supporting cellulolytic enzyme activity. The term is well known to a person skilled in the art. Preferred accessory proteins within the technical enzyme composition include Expansin, Swollenin, Loosenin and CIP Proteins (EC 3.1.1.-; CE15).

As used within the present invention, the term "oxidoreductase" refers to any enzyme capable of catalyzing an oxidation and/or a reduction reaction. Enzymes belonging to the class of oxidoreductases are classified as EC 1 in the EC number classification of enzymes. Oxidoreductase enzymes preferred within the technical enzyme composition include lytic polysaccharide monooxygenase (LPMO) (AA9-11; previously GH61 and CBM33, resp.) (EC 1.14.99.53-56, 1.14.99.B10), lignin peroxidase (EC 1.11.1.14), manganese peroxidase (EC 1.11.1.13), aryl-alcohol oxidase (EC 1.1.3.7), glyoxal oxidase (EC 1.1.3.), carbohydrate oxidases (EC 1.1.3.4, 9, 10), cellobiose dehydrogenase (EC 1.1.99.18), catalase (hydrogenperoxide oxidoreductase) (EC 1.11.1.6 or EC 1 .11.1.21), dye-decolorizing peroxidase (EC 1.11.1.19), laccase (EC 1.10.3.2), peroxidase (EC 1.11.1.x) and versatile peroxidase (EC 1.11.1.16).

As used within the present invention, the term "esterases" refers to any enzyme capable of cleaving an ester bond. Esterases preferred within the technical enzyme composition include acetyl esterases, glucuronoyl esterases, feruoyl esterases, lipases, cutinases and phospholipases.

As used within the present invention, the term "alpha-glucanases" refers to any enzyme capable of degrading alpha-linked oligo- and polysaccharides. Alpha-glucanases preferred within the technical enzyme composition include alpha-amylases, glucoamylases, pullulanases, dextranases, trehalases, lactases, invertases and maltases.

As used within the present invention, the term "phosphatase" refers to any enzyme capable of cleaving phosphoester bonds. Phosphatases preferred within the technical enzyme composition include phytases.

As used within the present invention, the term "isomerases" refers to any enzyme capable of transferring a chemical compound into an isomeric structure. Isomerases preferred within the technical enzyme composition include xylose isomerases, glucose isomerases and arabinose isomerases.

As used within the present invention, the term "proteases" refers to any enzyme capable of cleaving a peptide bond. Proteases preferred within the technical enzyme composition include serine proteases, threonine proteases, aspartic proteases, cysteine proteases, glutamic proteases and metalloproteases.

The enzymes referenced within the present invention are classified according nomenclatures that are either based on the International Union of Biochemistry and Molecular Biology's Enzyme Nomenclature and Classification (http://www.chem.qmul.ac.uk/iubmb/enzyme/) or on Carbohydrate-Active EnZYmes (http://www.cazy.org/) database.

According to the present invention the term "fermentation medium" is to be understood as referring to any fermentation medium known to a person skilled in the art as suitable for the inventive process. Within the process of the present invention, the fermentation medium contains from 5 to 450 g/L glucose, wherein glucose contents from 5 to 420 g/L, from 8 to 400 g/L and from 10 to 280 g/L are preferred. Further preferred ranges of glucose are from 10 to 450 g/L, from 40 to 400 g/L and from 50 to 350 g/L. Also preferred ranges of glucose are from 5 to 50 g/L, from 6 to 40 g/L or from 7 to 35 g/L and from 50 to 450 g/L, from 80 to 400 g/L and from 100 to 380 g/L. The glucose contained in the fermentation medium may originate from any source known to a person skilled in the art as suitable for the inventive process. Within a preferred embodiment, the glucose originates from corn, sugar cane or sugar beets, preferred sources are corn syrup, sugar cane or sugar beet molasses and mixtures thereof.

Within a preferred embodiment of the present invention, the fermentation medium further contains xylose and wherein the glucose to xylose ratio is selected from the range of from 1 to 3.5., such as a ratio selected from the range of from 1 to 3, from 1 to 2.8, of from 1 to 2.5 or of from 1 to 2.2. Further preferred ratios are 2.1, 2.0, 1.9 and 1.8.

Within an alternative preferred embodiment of the present invention, the fermentation medium further contains lactose and wherein the glucose to lactose ratio is selected from the range of from 1 to 10, such as a ratio selected from the range of from 1 to 9, from 1 to 8.5, of from 1 to 8 or of from 1 to 7. Further preferred ratios are 3, 4, 5 and 6.

Within a preferred embodiment of the present invention no gluco-oligosaccharides have been added to the fermentation medium and it is particularly preferred that the fermentation medium is free from gluco-oligosaccharides.

Within a preferred embodiment of the present invention no sophorose has been added to the fermentation medium and it is particularly preferred that the fermentation medium is free from sophorose.

Within another preferred embodiment of the present invention the fermentation medium contains less than 100 g/L cellulose and/or hemicellulose, preferably less than 80 g/L, more preferred less than 70 g/L, even more preferred less than 60 g/L, particularly preferred less than 50 g/L, and most preferred less than 40 g/L cellulose and/or hemicellulose. Within another preferred embodiment the fermentation medium of the present invention is free from hemicellulose. Within a further preferred embodiment of the present invention the cellulose content of the fermentation medium is selected from the range of from 0.01 g/L to 50 g/L, preferably from 0.1 to 40 g/L, further preferred of from 1 to 30 g/L and most preferred of from 1 to 20 g/L.

Within another preferred embodiment the fermentation medium has a nitrogen content of from 0.05 to 50.0 g/L. Preferred contents of nitrogen are selected from the range of from 0.1 to 45 g/L, from 0.3 to 40 g/l or from 0.5 to 30 g/L. In case of small scale fermentations with a total volume of fermentation medium of less than 100L (liters), such as from 0.1 to less than 100 L, the nitrogen content of the fermentation medium is preferably selected from the range of from 0.05 to 2 g/L, further preferred of from 0.3 to 1.2 g/L and most preferred of from 0.5 to 1.0 g/L. In a preferred embodiment, small scale fermentations are carried out in reactors which are not stirred and not aerated. In case of large scale fermentations with a total volume of the fermentation medium of at least 100 L, such as for example from 100 to 10000000 L, the nitrogen content of the fermentation medium is preferably selected from the range of from 2.0 to 50 g/L, further preferred of from 5.0 to 40 g/L and most preferred of from 7.5 to 15.0 g/L. In a preferred embodiment, large scale fermentations are carried out in reactors which are stirred and/or aerated, The nitrogen can be added in any form known to a person skilled in the art as suitable for the inventive purpose and may be added in form of ammonium sulfate, ammonia, urea, or in form of a complex nitrogen source such as soy meal, corn steep liquor, brewer's spent grains, wet distillers grains (WDG), dried distillers grains with solubles (DDGS), peptone, yeast extract or combinations thereof. In case a complex nitrogen source is used, the amount of the complex nitrogen source needed has to be calculated in alignment with the desired nitrogen content of the fermentation medium. The amount of nitrogen can be added by feeding or by adding the total amount to the fermentation medium at any time before or during step (a) and/or (b) of the inventive process. It is thereby preferred that the nitrogen is added as a 25% (wt.-/wt.) solution of ammonia or a 40 % (wt./wt.) solution of urea.

Within another preferred embodiment of the present invention, the fermentation medium contains from 0.5 to 80 wt.-% molasses, corn syrup or mixtures thereof, preferably from 5 to 75 wt.-%, from 15 to 70 wt.-%, from 25 to 65 wt.-%, from 35 to 60 wt.-% from 38 to 55 wt.-% or from 40 to 52 wt.-%.

Within a preferred embodiment of the inventive process the pH of the fermentation medium has been adjusted to a pH selected from the range of from pH 2.0 to pH 6.0, wherein ranges of from pH 3.0 to 5.5 and from pH 3.5 to 5.5 as well as from pH 3.5 to 5.0 are particularly preferred. The adjusting of the pH can be carried out by any means and method known to a person skilled in the art as suitable for the inventive purpose. Within the process of the present invention the pH is preferably adjusted by addition of an acid such as sulfuric acid or acetic acid, NaOH, H₃PO₄ or ammonia.

Within a preferred embodiment of the inventive process the fermentation medium has a potassium hydrogen phosphate content of from 0.5 to 10.0 g/L, a magnesium sulfate heptahydrate content of from 0.05 to 1 g/L, a calcium chloride dihydrate content of from 0.1 to 1 g/L, an ammonium sulfate content of from 1.5 to 4.5 g/L, an iron (II) sulfate heptahydrate content of from 0.005 to 0.1 g/L, a manganese sulfate content of from 0.00001 to 0.001 g/L, a zinc sulfate heptahydrate content of from 0.001 to 0.01 g/L and/or a copper sulfate pentahydrate content of from 0.0001 to 0.001 g/L. Further preferred ranges are potassium hydrogen phosphate content of from 1 to 8.0 g/L, a magnesium sulfate heptahydrate content of from 0.1 to 0.8 g/L, a calcium chloride dihydrate content of from 0.3 to 0.8 g/L, an ammonium sulfate content of from 1.7 to 4.0 g/L, an iron (II) sulfate heptahydrate content of from 0.01 to 0.9 g/L, a manganese sulfate content of from 0.0001 to 0.0008 g/L, a zinc sulfate heptahydrate content of from 0.002 to 0.008 g/L and/or a copper sulfate pentahydrate content of from 0.0002 to 0.008 g/L.

The "providing" of the fermentation medium according to step (a) of the inventive process can be carried out by any method and within any means known to a person skilled in the art as suitable for the inventive process. Within a preferred embodiment the fermentation medium is provided within a batch or fed batch reactor which is preferred equipped with a stirring device and a cooling device.

According to step (b) of the inventive process, at least one filamentous fungus cell wherein SEQ ID NO: 1 has been disrupted is added to the fermentation medium. The addition can be carried out by any means and measure known to a person skilled in the art as suitable for the inventive process. Within a preferred embodiment, the at least one filamentous fungus cell is added in a quantity of from 10² to 10¹⁰ cells, preferably in a quantity of from 10³ to 10⁸ cells and most preferred in a quantity of from 10⁴ to 10⁷ cells per g of fermentation medium. The at least one filamentous fungus cell can thereby be added in dried form, as conidia or in form of a preculture, containing rest of preculturing medium. It is also possible to add the at least one filamentous fungus cell in form of a fully cultured medium (also referred to as main culture).

Within the present invention the term "filamentous fungus cell" is to be understood as any cell from any filamentous fungus existing in nature and/or known to a person skilled in the art. The term also comprises any filamentous fungus cell either of natural origin or modified. The term "modified" refers to genetically and non-genetically modified fungi. i.e. fungi which have been modified by genetic methods (e.g. transformation) and non-genetic methods e.g. chemical mutagenesis or irradiation, both of which are known to those skilled in the art. Within a preferred embodiment the at least one filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes,* wherein *Trichoderma* and *Aspergillus* are particularly preferred, most preferred is *Trichoderma reesei* (teleomorph: *Hypocrea jecornia*).

It is another advantage of the present invention that in case the filamentous fungal cell is from the species *Trichoderma,* the *Trichoderma* cell produces an increased amount of at least one aspartate protease. Aspartate proteases play a significant role in breaking down complex nitrogen sources such as soy meal, corn steep liquor, brewer's spent grains, wet distillers grains (WDG), dried distillers grains with solubles (DDGS), yeast extract or peptone. Therefore, a high amount of aspartate protease(s) will enable the *Trichoderma* fungus to grow faster due to an increased availability of complex nitrogen compounds and to produce a higher amount of the technical enzyme composition within the production time. Further, a higher amount of those byproducts or waste products can be incorporated into the growth medium contributing to the sustainability of the inventive process. Within the state of the art no or only a limited amount could be used as a nitrogen source and further nitrogen had to be supplemented in form of chemically synthesized ammonia or urea. A suitable growth medium contains from 0.25 to 75 g/L of at least one complex nitrogen source selected from the group consisting of soy meal, corn steep liquor, brewer's spent grains, wet distillers grains (WDG), dried distillers grains with solubles (DDGS), yeast extract, peptone or mixtures thereof. The amount of complex nitrogen source is to be calculated in accordance with the above definitions and required nitrogen content of the fermentation medium.

Within another preferred embodiment of the present invention, the at least one filamentous fungus cell is a genetically modified filamentous fungus cell with the ability to express at least one heterologous hydrolyase or oxidoreductase enzyme, such as but not limited to an enzyme belonging to the class of cellulases, belonging to the class of beta-glucosidases or belonging to the class of xylanases or belonging to the class of lytic polysaccharide monooxygenases. Within such a preferred embodiment, the at least one heterologous hydrolase or oxidoreductase enzyme preferably originates from another filamentous fungus such as - but not limited to - *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.* Within a particularly preferred embodiment the at least one filamentous fungus cell is a *Trichoderma reesei* cell and the at least one heterologous hydrolase or oxidoreductase enzyme originates from *Acremonium, Ajellomyces, Alternaria, Armillaria, Arthroderma, Aspergillus, Bionectria, Bipolaris, Ceriporiopsis, Chaetomium, Cladophialophora, Clohesyomyces, Colletotrichum, Coniochaeta, Coniosporium, Diaporthe, Dothistroma, Emericella, Epicoccum, Exophiala, Fomes, Fonsecaea, Fusarium, Gibberella, Grosmannia, Hebeloma, Hortaea, Humicola, Hypocrea, Hypoxylon, Irpex, Isaria, Kuraishia, Leucoagaricus, Madurella, Magnaporthe, Marssonina, Metarhizium, Moniliophthora, Myceliophthora, Mycosphaerella, Neurospora, Oidiodendron, Ophiostoma, Paecilomyces, Paraphaeosphaeria, Penicillium, Phanerochaete, Phialophora, Pleurotus, Pochonia, Pseudocercospora, Pseudogymnoascus, Pyrenophora, Rasamsonia, Rhinocladiella, Rhizopus, Rhizosphaera, Rhynchosporium, Setosphaeria, Sphaerulina, Sporothrix, Stachybotrys, Stemphylium, Talaromyces, Termitomyces, Tilletiaria, Torrubiella, Trametes, Trichoderma, Trichophyton, Uncinocarpus* and/or *Valsa* species.

According to the present invention, the at least one filamentous fungus cell as is a filamentous fungus cell wherein SEQ ID NO: 1 has been disrupted. The "disruption" can thereby be carried out by any means and measure known to the person skilled in the art as suitable for the purpose of disruption. The term "disruption" comprises all techniques that either lead to the gene no longer being transcribed or to the protein encoded by the gene no longer being produced or only being produced in an inactive form.

Exemplary methods which can be used within the present invention are:
- the partial or complete removal from the genome of the gene, the region coding for the protein and/or the promoter or other regions necessary for the expression of the gene (= "deletion")
- the alteration of the DNA sequence of the coding region so that a shortened protein (= generation of a stop codon) and/or a protein with an altered amino acid sequence is produced which can no longer perform the function of the unchanged protein (= "mutation")
- the modification of the DNA sequence of the promoter or other regions necessary for the expression of the gene, so that the gene is no longer transcribed (= no RNA and therefore no protein is produced)
- the expression of RNA with a sequence complementary to that of the target gene. This leads to hybridization (= pairing of complementary sequences) of the two RNAs and to a degradation of this double-stranded RNA. As a result, no RNA of the target gene is available for protein synthesis (= RNA interference).

Within the present invention SEQ ID NO:1 is defined within the sequence protocol.

Mixing according to step (c) of the inventive process of the present invention is carried out for a time period from 1 minute to 10 days, preferably from 10 hours to 7 days, further preferred from 24 hours to 5 days, preferably under constant stirring with a power input from 150 to 20000 W/ m³ and more preferably from 500 to 15000 W/m³ and under oxygen controlled conditions. The average dissolved oxygen level is preferably selected from 0.01% to 80%, preferred from 0.1% to 50%, particularly preferred from 5% to 30% and most preferred from 12% to 28%. Within a particularly preferred embodiment, the dissolved oxygen level is controlled by a stirrer or compressed air flow or internal reactor pressure or a combination of two or three of these measures. Furthermore, mixing according to step (c) of the inventive process is carried out at a temperature of from 20 to 35 °C, preferably at a temperature of from 21 to 34 °C wherein a temperature selected from the range of from 22 to 33 °C is also preferred.

"Mixing" according to step (c) of the process of the present invention is preferably conducted in a batch mode (discontinuous), in a fed-batch mode or in a continuous mode. Most preferably, the inventive process is conducted in a fed-batch mode.

"Obtaining" according to step (d) of the inventive process is preferably carried out by harvesting the technical enzyme composition at the end of the time period applied for mixing during step (c) as it is without further treatment.

Within another preferred embodiment of the present invention, the inventive process further contains the step (e): subjecting the technical enzyme composition according to step d) to a purification method.The purification according to step (e) can be carried out by any measure known to a person skilled in the art as suitable for the inventive purpose. Suitable purification methods are selected from the group consisting of filtration (ultrafiltration, microfiltration, nanofiltration, depth filtration, sterile filtration, filter press), centrifugation, decantation, flotation, chromatographic separation, adsorption, electrodialysis, extraction, precipitation, crystallisation, spray drying, granulation, coating, extrusion or combinations thereof. Preferred are filter-based solid-liquid separations. It is further particularly preferred to use a filter press. The residues after the filtration should have a minimal solid content of 20 % (wt./wt.), preferably 25 % (wt./wt.), particularly preferred 30 % (wt./wt.) and most preferred 40 % (wt./wt.) solid content. In case the process according to the present invention involves solid-liquid separation as purification, the technical enzyme composition obtained according to step (d) of the inventive process is considered to be the liquid fraction.

Within a preferred embodiment of the inventive process, the process further comprises step

### (ai) sterilization of the fermentation medium according to step (a).

Sterilization can thereby be carried out by any means or measure known to a person skilled in the art as suitable for the inventive purpose. Within a preferred embodiment, sterilization is carried out by filtration, such as but not limited to membrane filtration processes or by ultra high temperature heating. A combination of two or more sterilization methods is also possible, however, it is particularly preferred to only apply ultra high temperature heating (also referred to as UHT). The UHT treatment is preferably carried out at a temperature of from 100 to 155 °C and for a duration of from 10 to 30 seconds, more preferred at a temperature of from 120 to 140 °C for a duration of from 10 to 20 seconds.

Within another aspect, the present invention relates to a filamentous fungus cell wherein SEQ ID NO:1 has been disrupted. Disruption of SEQ ID NO:1 can be carried out by any means and measure known to a person skilled in the art to be suitable for the inventive purpose. Possible and preferred methods and measures have been defined within the description. Within a preferred embodiment, SEQ ID NO:1 has been disrupted by deletion, mutation, modification of a promotor or any other regulatory sequence, generation of a stop codon or RNA interference. The term "filamentous fungus cell" has been defined within the description. All definitions given apply.

Within a preferred embodiment, the filamentous fungus cell is a genetically modified filamentous fungus cell with the ability to express at least one heterologous hydrolase enzyme. Such genetically modified filamentous fungus cell has been defined within the description. Within a particularly preferred embodiment of the present invention, the filamentous fungus cell is a genetically modified filamentous fungus cell wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase encoding sequence, at least one heterologous protease enzyme encoding sequence, at least one heterologous isomerase enzyme encoding sequence and/or at least one heterologous lytic polysaccharide monooxygenase enzyme encoding sequence.

In another aspect the present invention relates to a technical enzyme composition produced according to the process as defined before.

In a further aspect the present invention relates to the use of a filamentous fungus cell as defined before for the production of a technical enzyme composition as defined before.

### Generally preferred embodiments

In the following, generally preferred embodiments of the present invention are listed which do not limit the scope of the invention and/or scope of the claims in any respect. The generally preferred embodiments illustrate particularly suitable embodiments for the production of technical enzyme composition by the filamentous fungus *Trichoderma reesei.*

### Generally preferred embodiment 1

Process for production of a technical enzyme composition, comprising the following steps:
(a) providing a fermentation medium with a glucose content of from 5 to 450 g/L;
(b) addition of at least one *Trichoderma reesei* cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 2

Process for production of a technical enzyme composition, comprising the following steps:
(a) providing a fermentation medium with a glucose content of from 5 to 450 g/L and wherein the fermentation medium further contains xylose and wherein the glucose to xylose ratio is selected from the range of from 1 to 3.5;
(b) addition of at least one *Trichoderma reesei* cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 3

Process for production of a technical enzyme composition, comprising the following steps:
(a) providing a fermentation medium with a glucose content of from 5 to 450 g/L and wherein the fermentation medium is free from cellulose, hemicellulose, gluco-oligosaccharides and/or sophorose;
(b) addition of at least one *Trichoderma reesei* cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 4

Process for production of a technical enzyme composition, comprising the following steps:
(a) providing a fermentation medium with a glucose content of from 5 to 450 g/L and a cellulose content of from 0.01 g/L to 1 g/L;
(b) addition of at least one *Trichoderma reesei* cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 5

Process for production of a technical enzyme composition, comprising the following steps:
(a) providing a fermentation medium with a glucose content of from 5 to 450 g/L;
(b) addition of at least one *Trichoderma reesei* cell wherein SEQ ID NO:1 has been disrupted by deletion, mutation, modification of a promotor or any other regulatory sequence, generation of a stop codon or RNA interference and wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase encoding sequence, at least one heterologous protease enzyme encoding sequence, at least one heterologous isomerase enzyme encoding sequence and/or at least one heterologous lytic polysaccharide monooxygenase enzyme encoding sequence;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 6

Process for production of a technical enzyme composition, comprising the following steps:
(a) providing a fermentation medium with a glucose content of from 5 to 450 g/L and wherein the fermentation medium is free from sophorose and/or gluco-oligosaccharides;
(b) addition of at least one *Trichoderma reesei* cell wherein SEQ ID NO:1 has been disrupted by deletion, mutation, modification of a promotor or any other regulatory sequence, generation of a stop codon or RNA interference and wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase encoding sequence, at least one heterologous protease enzyme encoding sequence, at least one heterologous isomerase enzyme encoding sequence and/or at least one heterologous lytic polysaccharide monooxygenase enzyme encoding sequence;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 7

*Trichoderma reesei* cell, wherein SEQ ID NO:1 has been disrupted by deletion, mutation, modification of a promotor or any other regulatory sequence, generation of a stop codon or RNA interference, comprising at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase encoding sequence, at least one heterologous protease enzyme encoding sequence, at least one heterologous isomerase enzyme encoding sequence and/or at least one heterologous lytic polysaccharide monooxygenase enzyme encoding sequence.

### Generally preferred embodiment 8

*Trichoderma reesei* cell, wherein SEQ ID NO:1 has been disrupted by deletion, mutation, modification of a promotor or any other regulatory sequence, generation of a stop codon or RNA interference, comprising at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase enzyme encoding sequence, at least one heterologous protease enzyme encoding sequence, at least one heterologous isomerase enzyme encoding sequence and/or at least one heterologous lytic polysaccharide monooxygenase enzyme encoding sequence and wherein the at least one heterologous enzyme sequence originates from *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes .*

### Generally preferred embodiment 9

Technical enzyme composition produced according to a process as defined by any of generally preferred embodiment 1 to 8.

### Generally preferred embodiment 10

Use of a filamentous fungus cell as defined by any of generally preferred embodiments 8 or 9 for the production of a technical enzyme composition.

### Generally preferred embodiment 11

Process for production of a technical enzyme composition as defined by any of generally preferred embodiments 1 to 6, wherein the growth medium contains from 0.05 to 50 g/L nitrogen added in form of at least one complex nitrogen source selected from the group consisting of soy meal, corn steep liquor, brewer's spent grains, wet distillers grains (WDG), dried distillers grains with solubles (DDGS), yeast extract, peptone or mixtures thereof.

### Generally preferred embodiment 12

Process for production of a technical enzyme composition as defined by any of generally preferred embodiments 1 to 6 and 11, wherein the growth medium contains from 0.05 to 2 g/L nitrogen added in form of at least one complex nitrogen source selected from the group consisting of soy meal, corn steep liquor, brewer's spent grains, wet distillers grains (WDG), dried distillers grains with solubles (DDGS), yeast extract, peptone or mixtures thereof and wherein the fermentation medium is in the range of from 0.1 to less than 100L.

### Generally preferred embodiment 13

Process for production of a technical enzyme composition as defined by any of generally preferred embodiments 1 to 6 and 11, wherein the growth medium contains from 2 to 50 g/L nitrogen added in form of at least one complex nitrogen source selected from the group consisting of soy meal, corn steep liquor, brewer's spent grains, wet distillers grains (WDG), dried distillers grains with solubles (DDGS), yeast extract, peptone or mixtures thereof and wherein the fermentation medium is in the range of from 100 to 10000000 L.

SEQ ID NO: 1

ATGAGTAGAAACCGTCGAGAGTCCCAAAATATCTTGGAGTCACTGCATTC
AAGGTACGCGTGTATACGGCAAGTTCCACGGGCATATACAGCAAATTACC
ATATCCAAGTCCTTACATGGTAAACCCAATAGCAACCCTTTCTTTGCCCTT
CAGGTTGTCCCGTCTCCGCGATGGTTTCAGGGTAAGGTGACGAACGGTA
CAGTAACAAAGACTCCAAAGACGAGCCCACGATCGGTGGAAGCAATGCG
TCATGCTGAGTCATCGACGCCCCTCCGTGGGTAAACAGGCAATGCCCCG
CCAACAGCCGTGAGAAGCAAAATAACATCATGACAGCTTCCAGCGCCTTG
CTTTTGCTCTCCTGCACCGCTCCTCTCCCTCGTTGCAGCTATTCGCATTG
TCCTACTCGAGGCTCGACGGCGGCCCGGCGCCCAAAATGTCCACGTAC
GTGACGCTATCGTCTACATCTCTCTGACGCTCTATACCTTACCTTGTCTCG
TCTCCGTGTGTTCTTGTCTAACACGCTCACCTGCATCATGATGCACCTCA
CAGGCCAACCACCAATATCCTCAGCATCCCATTTCGCCGGTCGCTGCAC
CTGTCGCTGTCGACGACGATCCGACAGTACATCAACACCAAATATGACCA
GCACCCGGACATGTTCCAGTATGACCTCGAGGCCATCGATGCGCTGCGC
CGCGACGCCGTGAACGTGCGCGAGCCGCACCTGAGCGGCATCAAGAAG
CTGCAGGTGTACGCGGGCCAGCTGGTGTGGATTGGCGGCAAGTTTCCG
ATTGATGTGCGTAGACGAAAGACGAGTAGGGGGAGGAGCAGGAGAAACA
AGCGGACAAGATGCTGATGCTGCTAGATGAACAGATCGGCGCCGAGTTC
ACCTGGTACCCGGCCCTTGGCTACCACACCGACCGGCCGATGGCGCGC
AACAACCTCAAGTACGAGCTCATGAATGTCCTCTACAACCTCGCCGCCTT
GTACTCTCAGCTTGCCCTCAACACGCCCCGCGGCGATACCGAGGGTCTC
AAGTCCGCCGCCAACTACTTTTCCCTAGCCGCCGGCGTCCTCTCCCACA
TTCAGAAAGCCGTGCTTCCCGAGCTGCGCATGTCCGACCCGCCCGACGA
CATGGACCACAACACTCTCGAATCGCTGTTGCAGCTGTTTCTGGCACAGA
GCCAGGAGTGCTTCTGGCAGAAGGCAGTCATGGACGGTTACAAGGACGC
CTCGATCGCAAAGCTGGCTGCGAGGGTCTCTGACCTGTACAACCTGGCG
GCCGAGGCTGCGGTGAACAGCGAGGCCATTAGTAGTGCCTGGATACATC
ACATGAACGCGAAGCACCACCACTTTGCAGCAGCTGCCCAGTATCGTGC
TGCCTGCGATTGCTTGGAGAAGAGAAGGTACGGCGAGGAGATTGCGCG
GCTGAAAGATGCCGTCATCTGTGCTAATGACGGTATTAAGGAGGGCCGG
GTTGCCCCCTTGAACAAGACGGTCATGGAGGATTTACAGGCCTTGAAGC

GAAAGCTGGAAGAGGATCTGAAGAGGGCTGAGAAAGACAATGACCTCAT
CTTTCTTAGTACGTTGCTCCGCCTCGTCAACTTACGCAAAGATTGTCCCC
AAAGCTGACAGCCACCAACAGATCCTATACCCCCAAAGGCAGAACTGAA
GATCCTGGAGAGAGCCAACATGGCTGTTGCTCGAACGCCCCCCAGGTA
GCCAATCCGCTTGACTACCTAGGTGACCATGCCGAGCTTGGACCGGCAC
TGTTCTCTAAGCTGGTCCCGTTCTCGGTGCATGTTGCTATTTCCATCTAC
GAGGAGCGCAGAGATCGGCTGGTCAACCAAAACATCATTCAAGAGCTGG
AGAACCTGACCGACAAGATCCACACACTTCTCAGCTCTATTGGCTTGCCA
GGATCTTTGCAAGCGTTGGAGAAGCCTCTCGGCCTCCCACCTAGCTTGA
TACAACACGCGGAGGAGATTCGACAAGCTGACGCGATCAACAAGATCCA
GAGGAGCTTCGCCGACATCGAAAAGCTGCGGGCCAACGACTGGGCGAT
TTTCGAGGAGGGAAAAGCAGCGCTGGCCGCTGAAGAGGAGGAAGACGA
GCAGCTACGGAGGAAATACGGCACCAGCCGTTGGCGGCGCCCCGAGAG
CCAAGCAGACCCCAACGGCGCGAAGTTCTGGGCCGCCATTAACGAGATA
GGAGGCTATTTCCAGAATAGCGCAAGTAGCGACGAGGCGGTTCGAGACA
AGTTCATGGCGAACAAAGATTTGTTGGAGATCCTGTCAGGGTCAAACCAG
TCTCTGATGAACTACGTGCCCTCGAGCGCCCCCGTGGAAACCTCGGGTG
ACCTCAAGGCAGCTGTTGGGCGGTTGCGGAGCGTGTACAATGATGTTCT
GCGGATGGAGAGTAGGAGGAGGAAAAAGGCTGAGAGCCTGAGGGAGGC
AGCGCGGCGCGATGACATCAAGCCCGATATTCTCAAGGAGGCGGCTCG
CCTGGAGCGAGCATATCCCTCAACGCCTCTGCAGACAGTTCACTTTGAG
GAGTTTTTCGAAAAGCGACTGGATAAGCTGTACGAGCCAGAGCTCGAGG
CCGTCGAAAAGGAAGCACAGGACCAAGAGAATCTGCTGACCCTGCTAGA
GCGCGCAAACAGGGAGTTTGAGGCTCAGAAGCGCCTCATTGACGCCAAA
GGGCACCGTGATCGCGAGCAAGTGCTGCAGAAGCTCAATGGCGCGTACT
TCAAGTACAAGGAGATTGTGGCCAACCTGGAGGTGGGAGAAAGTTCTA
TAACGACCTGAATAGGATAGTTGCACATGGCTTCCGTGATGCCGTCAAAG
CATGGGTGGCGGAGCGGCGACTCGAGGCCAAGAGACTGGAAGAGTATG
TTGTTTGCTTGGTAAAAAGCTCCATATCGGACTCCTTGCTGACGCTGTCC
TAGGGAACTTAATATGCCGCCGCTCTCGGCTCTCAACATCAACCATCCGC
AGCCTGTTCAAAACCCACCATCCGGTTTCGACGCTCAGCCTGTGGCTCA
CCAACCTGTCCAGCAGCTACATGACCAATACCAGCCTGCATACCAGCAG
CAGACCTACCAGCAACCCTCATATCAACAGCAGCCGCTGCAAGCACAAC
AACAGTATCATCAGCCACAGCCAACACCACAACAACAGCCTGTCTATGCC

AGACAGGCCGTTCAGAGTCCGGCCGAGGCTTCAATACAATCGTGGGCCG
GAGGCCAAACGCAGCC GCCACTTCCGCAACAGAAACCGTCACAGCCTGG
GCAACAACCAAATCAATCGGCTGGAACGTGGAATCCTGCCATGGGCATC
AAGTTTGGAGGGCCATCGGCTGGTGGATCGTCTGGTCAGGAAGGAACAT
GGACCGCCGGTTCAGGGATTAGATTTGGCTGA

## Claims

1. Process for production of a technical enzyme composition, comprising the following steps:
(a) providing a fermentation medium with a glucose content of from 5 to 450 g/L;
(b) addition of at least one filamentous fungus cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

2. Process according to claim 1, wherein the pH of the fermentation medium according to step (a) has been adjusted to a pH selected from pH 2.0 to 6.0.

3. Process according to any of the foregoing claims, wherein the fermentation medium further contains xylose and wherein the glucose to xylose ratio is selected from the range of from 1 to 3.5.

4. Process according to any of claims 1 or 2 wherein the fermentation medium further contains lactose and wherein the glucose to lactose ratio is selected from the range of from 1 to 10.

5. Process according to any of the foregoing claims, wherein no gluco-oligosaccharides have been added to the fermentation medium.

6. Process according to any of the foregoing claims, wherein no sophorose has been added to the fermentation medium.

7. Process according to any of the foregoing claims, further comprising step (ai) sterilization of the fermentation medium according to step (a).

8. Process according to any of the foregoing claims, wherein the fermentation medium has a potassium hydrogen phosphate content of from 0.5 to 10.0 g/L, a magnesium sulfate heptahydrate content of from 0.05 to 1 g/L, a calcium chloride dihydrate content of from 0.1 to 1 g/L, an ammonium sulfate content of from 1.5 to 4.5 g/L, an iron (II) sulfate heptahydrate content of from 0.005 to 0.1 g/L, a manganese sulfate content of from 0.00001 to 0.001 g/L, a zinc sulfate heptahydrate content of from 0.001 to 0.01 g/L and/or a copper sulfate pentahydrate content of from 0.0001 to 0.001.

9. Process according to any of the foregoing claims wherein the fermentation medium has a nitrogen content of from 0.05 to 50.0 g/L.

10. Process according to any of the foregoing claims, wherein the filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.*

11. Process according to any of the foregoing claims, wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidoreductase encoding sequence, at least one heterologous protease enzyme encoding sequence, at least one heterologous isomerase enzyme encoding sequence and/or at least one heterologous lytic polysaccharide monooxygenase enzyme encoding sequence.

12. Process according to any of the foregoing claims further comprising the step (e) subjecting the technical enzyme composition according to step d) to a purification method.

13. Process according to any of the foregoing claims wherein the filamentous fungus cell is a Trichoderma filamentous fungus cell and wherein the fermentation medium contains from 0.05 to 50 g/L nitrogen added in form of a complex nitrogen source selected from the group consisting of soy meal, corn steep liquor, brewer's spent grains, wet distillers grains (WDG), dried distillers grains with solubles (DDGS), yeast extract, peptone or mixtures thereof.

14. Filamentous fungus cell wherein SEQ ID NO:1 has been disrupted.

15. Filamentous fungus cell according to claim 14, wherein SEQ ID NO:1 has been disrupted by deletion, mutation, modification of a promotor or any other regulatory sequence, generation of a stop codon or RNA interference.

16. Filamentous fungus cell according to any of claims 14 or 15, wherein the at least one filamentous fungus cell is a genetically modified filamentous fungus cell wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidoreductase encoding sequence, at least one heterologous protease enzyme encoding sequence, at least one heterologous isomerase enzyme encoding sequence and/or at least one heterologous lytic polysaccharide monooxygenase enzyme encoding sequence.

17. Technical enzyme composition produced according to a process as defined in any of claims 1 to 13.

18. Use of a filamentous fungus cell as defined in any of claims 14 to 16 for the production of a technical enzyme composition.


SEQUENCE LISTING

<110> Clariant Produkte (Deutschland) GmbH

<120> PROCESS FOR THE PRODUCTION OF A TECHNICAL ENZYME COMPOSITION WITH LOW VISCOSITY PRODUCED BY A FILAMENTOUS FUNGUS

<130> 2021DE609-EP-EPA

<160> 1

<170> BiSSAP 1.3.6

<210> 1
<211> 3422
<212> DNA
<213> Trichoderma reesei

<220>
<223> native gene

<400> 1
atgagtagaa accgtcgaga gtcccaaaat atcttggagt cactgcattc aaggtacgcg 60

tgtatacggc aagttccacg ggcatataca gcaaattacc atatccaagt ccttacatgg 120

taaacccaat agcaaccctt tctttgccct tcaggttgtc ccgtctccgc gatggtttca 180

gggtaaggtg acgaacggta cagtaacaaa gactccaaag acgagcccac gatcggtgga 240

agcaatgcgt catgctgagt catcgacgcc cctccgtggg taaacaggca atgccccgcc 300

aacagccgtg agaagcaaaa taacatcatg acagcttcca gcgccttgct tttgctctcc 360

tgcaccgctc ctctccctcg ttgcagctat tcgcattgtc ctactcgagg ctcgacggcg 420

gcccggcgcc caaaatgtcc acgtacgtga cgctatcgtc tacatctctc tgacgctcta 480

taccttacct tgtctcgtct ccgtgtgttc ttgtctaaca cgctcacctg catcatgatg 540

cacctcacag gccaaccacc aatatcctca gcatcccatt tcgccggtcg ctgcacctgt 600

cgctgtcgac gacgatccga cagtacatca acaccaaata tgaccagcac ccggacatgt 660

tccagtatga cctcgaggcc atcgatgcgc tgcgccgcga cgccgtgaac gtgcgcgagc 720

cgcacctgag cggcatcaag aagctgcagg tgtacgcggg ccagctggtg tggattggcg 780

gcaagtttcc gattgatgtg cgtagacgaa agacgagtag ggggaggagc aggagaaaca 840

agcggacaag atgctgatgc tgctagatga acagatcggc gccgagttca cctggtaccc 900

ggcccttggc taccacaccg accggccgat ggcgcgcaac aacctcaagt acgagctcat 960

gaatgtcctc tacaacctcg ccgccttgta ctctcagctt gccctcaaca cgccccgcgg 1020

cgataccgag ggtctcaagt ccgccgccaa ctacttttcc ctagccgccg gcgtcctctc 1080

ccacattcag aaagccgtgc ttcccgagct gcgcatgtcc gacccgcccg acgacatgga 1140

ccacaacact ctcgaatcgc tgttgcagct gtttctggca cagagccagg agtgcttctg 1200

gcagaaggca gtcatggacg gttacaagga cgcctcgatc gcaaagctgg ctgcgagggt 1260

ctctgacctg tacaacctgg cggccgaggc tgcggtgaac agcgaggcca ttagtagtgc 1320

ctggatacat cacatgaacg cgaagcacca ccactttgca gcagctgccc agtatcgtgc 1380

tgcctgcgat tgcttggaga agagaaggta cggcgaggag attgcgcggc tgaaagatgc 1440

cgtcatctgt gctaatgacg gtattaagga gggccgggtt gcccccttga acaagacggt 1500

catggaggat ttacaggcct tgaagcgaaa gctggaagag gatctgaaga gggctgagaa 1560

agacaatgac ctcatctttc ttagtacgtt gctccgcctc gtcaacttac gcaaagattg 1620

tccccaaagc tgacagccac caacagatcc tataccccca aaggcagaac tgaagatcct 1680

ggagagagcc aacatggctg ttgctcgaac gcccccccag gtagccaatc cgcttgacta 1740

cctaggtgac catgccgagc ttggaccggc actgttctct aagctggtcc cgttctcggt 1800

gcatgttgct atttccatct acgaggagcg cagagatcgg ctggtcaacc aaaacatcat 1860

tcaagagctg gagaacctga ccgacaagat ccacacactt ctcagctcta ttggcttgcc 1920

aggatctttg caagcgttgg agaagcctct cggcctccca cctagcttga tacaacacgc 1980

ggaggagatt cgacaagctg acgcgatcaa caagatccag aggagcttcg ccgacatcga 2040

aaagctgcgg gccaacgact gggcgatttt cgaggaggga aaagcagcgc tggccgctga 2100

agaggaggaa gacgagcagc tacggaggaa atacggcacc agccgttggc ggcgccccga 2160

gagccaagca gaccccaacg gcgcgaagtt ctgggccgcc attaacgaga taggaggcta 2220

tttccagaat agcgcaagta gcgacgaggc ggttcgagac aagttcatgg cgaacaaaga 2280

tttgttggag atcctgtcag ggtcaaacca gtctctgatg aactacgtgc cctcgagcgc 2340

ccccgtggaa acctcgggtg acctcaaggc agctgttggg cggttgcgga gcgtgtacaa 2400

tgatgttctg cggatggaga gtaggaggag gaaaaaggct gagagcctga gggaggcagc 2460

gcggcgcgat gacatcaagc ccgatattct caaggaggcg gctcgcctgg agcgagcata 2520

tccctcaacg cctctgcaga cagttcactt tgaggagttt ttcgaaaagc gactggataa 2580

gctgtacgag ccagagctcg aggccgtcga aaaggaagca caggaccaag agaatctgct 2640

gaccctgcta gagcgcgcaa acagggagtt tgaggctcag aagcgcctca ttgacgccaa 2700

agggcaccgt gatcgcgagc aagtgctgca gaagctcaat ggcgcgtact tcaagtacaa 2760

ggagattgtg gccaacctgg aggtggggag aaagttctat aacgacctga ataggatagt 2820

tgcacatggc ttccgtgatg ccgtcaaagc atgggtggcg gagcggcgac tcgaggccaa 2880

gagactggaa gagtatgttg tttgcttggt aaaaagctcc atatcggact ccttgctgac 2940

gctgtcctag ggaacttaat atgccgccgc tctcggctct caacatcaac catccgcagc 3000

ctgttcaaaa cccaccatcc ggtttcgacg ctcagcctgt ggctcaccaa cctgtccagc 3060

agctacatga ccaataccag cctgcatacc agcagcagac ctaccagcaa ccctcatatc 3120

aacagcagcc gctgcaagca caacaacagt atcatcagcc acagccaaca ccacaacaac 3180

agcctgtcta tgccagacag gccgttcaga gtccggccga ggcttcaata caatcgtggg 3240

ccggaggcca aacgcagccg ccacttccgc aacagaaacc gtcacagcct gggcaacaac 3300

caaatcaatc ggctggaacg tggaatcctg ccatgggcat caagtttgga gggccatcgg 3360

ctggtggatc gtctggtcag gaaggaacat ggaccgccgg ttcagggatt agatttggct 3420

ga 3422